# NEUE EUROPÄISCHE PATENTSCHRIFT

(11) **EP 1 404 286 B2**
(45) Veröffentlichungstag und Bekanntmachung des Hinweises auf die Entscheidung über den Einspruch: **09.12.2015**
(45) Hinweis auf die Patenterteilung: 16.08.2006
(21) Anmeldenummer: 02716801.2
(22) Anmeldetag: 22.02.2002
(51) Int. Cl.: A61K 8/41, A61K 8/365, A61Q 7/00

(54) **HAARBEHANDLUNGSMITTEL MIT CARNITINTARTRAT**
HAIR TREATMENT AGENT WITH CARNITINE TARTRATE
AGENT DE SOIN CAPILLAIRE COMPRENANT UN TARTRATE DE CARNITINE

(30) Priorität: 19.03.2001 DE 10113446
(43) Veröffentlichungstag der Anmeldung: 07.04.2004
(60) Teilanmeldung: 06011936.9
(73) Patentinhaber: Henkel AG & Co. KGaA, 40589 Düsseldorf (DE)
(72) Erfinder: MÜLLNER, Stefan, 40764 Langenfeld (DE); HOTING, Edo, 22607 Hamburg (DE); PAUS, Ralf, 22453 Hamburg (DE)
(86) Internationale Anmeldenummer: PCT/EP2002/001875
(87) Internationale Veröffentlichungsnummer: WO 2002/074265

(56) Entgegenhaltungen:
- EP-A- 0 008 171
- EP-A- 1 175 887
- EP-A1- 0 434 088
- EP-A2- 0 688 555
- WO-A-02/24189
- WO-A-97/02041
- WO-A1-98/34610
- WO-A2-00/61543
- DE-A- 1 617 477
- DE-A1- 19 631 685
- US-A- 3 852 210
- US-A- 5 116 606
- US-A- 5 470 876
- PATENT ABSTRACTS OF JAPAN vol. 013, no. 501 (C-652), 10. November 1989 (1989-11-10) & JP 01 199905 A (FUJIO MORIKAWA), 11. August 1989 (1989-08-11)
- PATENT ABSTRACTS OF JAPAN vol. 2000, no. 04, 31. August 2000 (2000-08-31) & JP 2000 016920 A (KANEBO LTD;HANAOKA SHUSUKE), 18. Januar 2000 (2000-01-18)
- PATENT ABSTRACTS OF JAPAN vol. 1998, no. 01, 30. Januar 1998 (1998-01-30) & JP 09 241137 A (HOYU CO LTD), 16. September 1997 (1997-09-16)
- PATENT ABSTRACTS OF JAPAN vol. 1996, no. 09, 30. September 1996 (1996-09-30) & JP 08 127520 A (HASUNUMA KYOTARO;HANAOKA SHUSUKE), 21. Mai 1996 (1996-05-21)
- INTERNET ARTICLE, [Online] XP002206278 Gefunden im Internet: <URL:http://www.hairvitamins.net/viviscal/ vivlotion.htm> [gefunden am 2002-07-15] -& DATABASE PROMT [Online] 8. Februar 1999 (1999-02-08) "Viviscal Natural Food Supplement Tablets; Scalp Lotion" Database accession no. 1999:69163 XP002206289
- Testergebnisse: Wirkungsnachweis zur das Haarwachstum fördernden Wirkung von Carnitintartrat

## Beschreibung

In fast allen Kulturen umfaßt das propagierte und angestrebte Ideal für das äußere Erscheinungsbild der Menschen als wesentlichen Punkt ein volles und gepflegtes Haupthaar. Daher wird ein vorzeitiger Verlust der Haare, wie er meist aufgrund genetischer Prädisposition, aber auch durch Krankheit, Medikamenteneinfluß oder psychosomatische Gründe auftritt, von der überwiegenden Zahl der Menschen als Mangel empfunden. Es hat daher nicht an Versuchen gefehlt, Mittel bereitzustellen, die einem Haarausfall entgegenwirken bzw. stärkeren oder neuen Haarwuchs stimulieren. Daher sind eine Reihe von Haarwuchsmitteln auf dem Markt, deren Wirksamkeit aber mehr als umstritten ist.

Ursache für diesen Mißstand ist ein nur sehr lückenhaftes Wissen über den biologischen Mechanismus des Haarwuchses. So gibt es nur wenige empirische Aussagen über Wirkprinzipien; Ansätze zu einer übergreifenden Theorie des Haarwuchses fehlen fast vollständig. In der EP-A-102 534 wird beschrieben, daß Carbonsäuren mit einer ungeraden Anzahl von Kohlenstoffatomen und eine Reihe von Derivaten dieser Carbonsäuren sich durch eine bemerkenswert hohe haarwuchsstimulierende Wirkung auszeichnen.

K. Oba konnte in Tierversuchen nachweisen (Cosmetics & Toiletries, 103, 69 (1988)), daß den Haar- Follikeln durch das Aufbringen von Pentadecansäure- Glyzerin- Estern auf die Haut Energie zugeführt wird. Wird die übliche Energiezufuhr aufgrund der Inhibierung der Phosphofructokinase gestört oder fällt ganz aus, wie es bei der männlichen Glatze geschieht, so stehe mit den genannten Lipiden eine alternative Energiequelle zur Verfügung.

Es wurde nun überraschenderweise gefunden, daß bei Applikation von bestimmten Betainen das Haarwachstum signifikant verbessert wird.

Gegenstand der Erfindung ist somit ein kosmetisches Verfahren zur Verbesserung des Haarwuchses, dadurch gekennzeichnet, dass die Haare mit einem Mittel, enthaltend übliche Träger-, Wirk-, und Hilfsstoffe, wobei diese Mittel als Wirkstoff ein Mischsalz aus einem Betain der Formel (A-I) und Weinsäure enthalten, behandelt wird. Ein weiterer Gegenstand der Erfindung ist die Verwendung von Mischsalzen aus Carnitin und Weinsäure zur Herstellung eines pharmazeutischen Mittels zur Förderung des Wachstumes und/oder Verhinderung des Ausfallens von Haaren.

Hier sind unter den erfindungsgemäßen Betainen solche zu verstehen, weiche der Formel (A-I) entsprechen.

R¹R²R³N⁺-(CR⁴R⁵)ₓ-(CR⁶R⁷)_{y}-(CR⁸R⁹)_{z}-Y⁻ (A-I)

Der erfindungsgemäße Wirkstoff der Formel (A-I) ist Carnitin.

Unter Mischsalzen sind feste Lösungen verschiedener Substanzen zu verstehen. Zur allgemein anerkannten Definition von Mischkristallen als feste Lösungen sei beispielsweise auf H.R. Christen, Grundlagen der allgemeinen und anorganischen Chemie, Sauerländer und Salle, 5. Auflage, 1977, auf Seite 245 verwiesen und ausdrücklich Bezug genommen. Weiterhin werden die unterschiedlichen Arten der Mischkristallbildung, wie beispielsweise Isomorphie, Homöomorphie, Heteromorphie, statistische Mischkristallbildung auch Doppelsalzbildung genannt, Mischkristalle mit oder ohne Mischungslücke etc., gemäß der Definition aus Hollemann - Wiberg, Lehrbuch der anorganischen Chemie, Walter de Gruyter, 81. - 90. Auflage 1976, Kapitel VII, Die chemische Bindung unter cc) Die Mischkristallbildung auf Seite 114 beschrieben, erfindungsgemäß unter den Mischkristallen der erfindungsgemäßen Wirkstoffe der Formel (A-I) verstanden. Auf diese Definition wird ebenfalls ausdrücklich Bezug genommen.

Erfindungsgemäß kann es bevorzugt sein, das Mischsalz in fester Form in die Formulierungen einzuarbeiten. Es ist jedoch selbstverständlich auch möglich, das Mischsalz in Form ihrer einzelnen Komponenten zu verwenden. Zur Herstellung der Mischsalze der erfindungsgemäßen Wirkstoffe der Formel (A-I) sei beispielhaft auf die US - Patentschrift 5,073,376, die europäische Anmeldeschrift EP 0 434 088 A1 oder die US Patentschrift 5,071,874 verwiesen. Das Mischungsverhältniss der erfindungsgemäßen Mischsalze kann dabei bezogen auf die jeweiligen Molmassen der einzelnen Komponenten (erfindungsgemäßes Betain der Formel (A-I) / Mischsalz bildende Substanz) zwischen 1: 50 und 50 : 1, bevorzugt zwischen 10 : 1 und 1 : 10 und ganz besonders bevorzugt zwischen 3 : 1 und 1 : 3 betragen.

Es können erfindungsgemäß alle Arten von Isomeren, wie beispielsweise Diastereomere, Enantiomere, cis - trans- Isomere, optische Isomere, Konformationsisomere und Racemate verwendet werden.

Die Zusammensetzungen für das erfindungsgemäße kosmetische Verfahren gemäß Anspruch 1 und die erfindungsgemäße Verwendung gemäß Anspruch 12 enthalten die Betaine in Mengen von 0,001 bis 20 Gew.%, bezogen auf das gesamte Mittel. Ein Gehalt von 0,05 bis 10 Gew.% ist bevorzugt.

Die Zusammensetzungen für das erfindungsgemäße kosmetische Verfahren gemäß Anspruch 1 und die erfindungsgemäße Verwendung gemäß Anspruch 12 können die Betaine als einzige pharmakologisch und/oder kosmetisch wirksame Substanzen enthalten. Es ist jedoch auch möglich, den Mitteln weitere pharmakologisch und/oder kosmetisch wirksame Substanzen zuzusetzen.

Beispiele für pharmakologisch wirksame Substanzen sind Corticosteroide, β- Blocker, Östrogene, Cyproteronacetat, vasodilatorisch wirkende Substanzen wie Diazoxid, Nifedipin und Minoxidil.

Die Zusammensetzungen für das erfindungsgemäße kosmetische Verfahren gemäß Anspruch 1 und die erfindungsgemäße Verwendung gemäß Anspruch 12 können reine Wirkstofflösungen in einem geeigneten Lösungsmittel sein. Als Lösungsmittel können erfindungsgemäß Wasser oder Alkohole oder Mischungen daraus verwendet werden. Unter

Alkoholen sind gemäß einer ersten Ausführungsform lineare, verzweigte oder cyclische, gesättigte oder ungesättigte Alkohole mit 1 bis 12 Kohlenstoffatomen zu verstehen. Beispielhaft seien hierfür Methanol, Ethanol, Propanole, Butanole, Pentanole, Hexanole, Cyclohexanole, Heptanole, Octanole, Nonanole, Decanole, Undecanole sowie Dodecanole genannt.

Gemäß einer weiteren Ausführungsform handelt es sich bei den Löungsmitteln um Polyole. Geeignete Polyole sind beispielsweise Glycerin und Partialglycerinether, 2- Ethyl- 1,3- hexandiol, 1,3- Butandiol, 1,4- Butandiol, 1,2- Propandiol, 1,3- Propandiol, Pentandiole, beispielsweise 1,2-Pentandiol, Hexandiole, beispielsweise 1,2- Hexandiol oder 1,6-Hexandiol, Dodekandiol, insbesondere 1,2- Dodekandiol, Neopentylglykol und Ethylenglykol. 1,3-Diole, insbesondere 2- Ethyl- 1,3- hexandiol und 1,3- Butandiol, haben sich als besonders gut geeignet erwiesen.

Diese Polyole sind in den Zusammensetzungen für das erfindungsgemäße kosmetische Verfahren gemäß Anspruch 1 und die erfindungsgemäße Verwendung gemäß Anspruch 12 bevorzugt in Mengen von 2 - 10 Gew.% bezogen auf das gesamte Mittel enthalten. Erfindungsgemäß können selbstverständlich auch mit Wasser nur begrenzt mischbare Alkohole eingesetzt werden.

Unter "mit Wasser begrenzt mischbar" werden solche Alkohole verstanden, die in Wasser bei 20 °C zu nicht mehr als 10 Gew.- %, bezogen auf die Wassermasse, löslich sind.

In vielen Fällen haben sich Triole und insbesondere Diole als erfindungsgemäß besonders geeignet erwiesen. Gemäß einer weiteren Ausführungsform der Erfindung einsetzbar sind Alkohole mit 4 bis 20, insbesondere 4 bis 10, Kohlenstoffatomen. Die verwendeten Alkohole können dann gesättigt oder ungesättigt und linear, verzweigt oder cyclisch sein. Beispiele sind Butanol- 1, Cyclohexanol, Pentanol- 1, Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Bei den Zusammensetzungen für das erfindungsgemäße kosmetische Verfahren gemäß Anspruch 1 und die erfindungsgemäße Verwendung gemäß Anspruch 12, enthaltend den Wirkstoff (A) kann es sich aber auch um übliche Haut- und Haarbehandlungsmittel handeln.

In diesen Fällen können die Zusammensetzungen für das erfindungsgemäße kosmetische Verfahren gemäß Anspruch 1 und die erfindungsgemäße Verwendung gemäß Anspruch 12, neben den genannten Betainen alle auf diesen Gebieten bekannten und üblicherweise verwendeten Träger,- Wirk- und Hilfsstoffe enthalten.

Solche Haut- und Haarbehandlungsmittel sind beispielsweise Shampoos, Haarnachspülmittel, Haargele, Haarwässer, Haarkuren, Haarcremes, Haarlotionen, Haarsprays und Haartinkturen. Die Anwendung dieser Mittel erfolgt dabei üblicherweise topisch.

Diese Zusammensetzungen für das erfindungsgemäße kosmetische Verfahren gemäß Anspruch 1 und die erfindungsgemäße Verwendung gemäß Anspruch 12 können in allen für Haut- und Haarbehandlungen üblichen Konfektionierungen, wie beispielsweise in Form einer wässrigen Lösung oder Emulsionen, wie beispielsweise einer O/W- oder W/O-Emulsion, welche nach der Phaseninversionstemperaturmethode hergestellt sein können, als Mikro- oder Nanoemulsion, einer wässrig-alkoholischen oder alkoholischen Lösung, einer Creme, eines Gels, einer Lotion oder eines Aerosoles vorliegen. Die Zusammensetzungen für das erfindungsgemäße kosmetische Verfahren gemäß Anspruch 1 und die erfindungsgemäße Verwendung gemäß Anspruch 12 mit dem Wirkstoff (A) können einphasig oder mehrphasig vorliegen.

Wenngleich die zuvor genannten Haut- und Haarbehandlungsmittel erfindungsgemäß bevorzugt sind, können die Betaine auch anderen Haarbehandlungsmitteln, wie z.B. Haarfärbemitteln und Wellmitteln, zugefügt werden. Diese Mittel enthalten dann gegebenenfalls die bekannten direktziehenden Farbstoffe, Vorläufer für Oxidationsfarbstoffe (Entwickler-und Kupplerkomponenten) und Oxidationsmittel bzw. Reduktionsmittel.

Die Zusammensetzungen für das erfindungsgemäße kosmetische Verfahren gemäß Anspruch 1 und die erfindungsgemäße Verwendung gemäß Anspruch 12 weisen bevorzugt einen pH-Wert von 2 bis 10, insbesondere von 4 bis 9, auf.

Entsprechend der Art des Haut- und Haarbehandlungsmittels und der gewählten Konfektionierungsform können die Zusammensetzungen für das erfindungsgemäße kosmetische Verfahren gemäß Anspruch 1 und die erfindungsgemäße Verwendung gemäß Anspruch 12 bevorzugt folgende weitere Inhaltsstoffe enthalten:
Als weitere Haut und Haar pflegende Substanzen können zusätzlich in den Zusammensetzungen für das erfindungsgemäße kosmetische Verfahren gemäß Anspruch 1 und die erfindungsgemäße Verwendung gemäß Anspruch 12 Fettstoffe (D) eingesetzt werden. Unter Fettstoffen sind zu verstehen Fettsäuren, Fettalkohole, natürliche und synthetische
   Wachse, welche sowohl in fester Form als auch flüssig in wäßriger Dispersion vorliegen können, und natürliche und synthetische kosmetische ölkomponenten zu verstehen.

Als Fettsäuren (D1) können eingesetzt werden lineare und/ oder verzweigte, gesättigte und/ oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Iso-palmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew. %, bezogen auf das gesamte Mittel. Bevorzugt beträgt die Menge 0,1 -10 Gew. %, wobei ganz besonders vorteilhaft Mengen von 0,1 - 5 Gew. % sein können.

Als Fettalkohole (D2) können eingesetzt werden gesättigte, ein- oder mehrfach ungesättigte, verzweigte oder unverzweigte Fettalkohole mit C6-C30-, bevorzugt C10-C22-und ganz besonders bevorzugt C12-C22- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer
Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette® , z.B. Lanette® O oder Lorol®, z.B. Lorol® C8, Lorol® C14, Lorol® C18, Lorol® C8-18, HD- Ocenol®, Crodacol® , z.B. Crodacol® CS, Novol® , Eutanol® G, Guerbitol® 16, Guerbitot® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden. Die Fettalkohole werden in Mengen von 0,1 - 30 Gew.- %, bezogen auf die gesamte Zubereitung, bevorzugt in Mengen von 0,1-20 Gew.- % eingesetzt.

Als natürliche oder synthetische Wachse (D3) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1-50 Gew. % bezogen auf das gesamte Mittel, bevorzugt 0,1 - 20 Gew. % und besonders bevorzugt 0,1-15 Gew. % bezogen auf das gesamte Mittel.

Zu den natürlichen und synthetischen kosmetischen Olkörpem (D4), welche erfindungsgemäß vorteilhaft verwendet werden können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di- n-alkylether mit insgesamt zwischen
   12 bis 36 C- Atomen, insbesondere 12 bis 24 C- Atomen, wie beispielsweise Di- n-octylether, Di- n- decylether, Di- n- nonylether, Di- n- undecylether, Di- n- dodecylether, n-Hexyl- n- octylether, n- Octyl- n- decylether, n- Decyl n- undecylether, n- Undecyl- n-dodecylether und n- Hexyl- n- Undecylether sowie Di- tert- butylether, Di- iso- pentylether, Di- 3-ethyldecylether, tert.-Butyl- n- octylether, isoPentyl- n- octylether und 2- Methylpentyl- n- octylether. Die als Handelsprodukte
   erhältlichen Verbindungen 1,3- Di-(2- ethyl- hexyl)-cyclohexan (Cetiol® S) und Di- n-octylether (Cetiol® OE) können bevorzugt sein.
- Esteröle. Unter Esterölen sind zu verstehen die Ester von C6-C30 - Fettsäuren mit C2-C30 - Fettalkoholen. Bevorzugt sind die Monoester der Fettsäuren mit Alkoholen mit 2 bis 24 C- Atomen. Beispiele für eingesetzte Fettsäurenanteile in den Estern sind Capronsäure, Caprylsäure, 2- Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2- Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2- ethylhexylester (Cetiol® 868), Cetyloleat, Glycerintricaprylat, Kokosfettalkohol- caprinat/- caprylat (Cetiole LC), n-Butylstearat, Oleylerucat (Cetiol® J 600), Isopropylpalmitat (Rilanit® IPP), Oleyl Oleate (Cetiol® ), Laurinsäurehexylester (Cetiol® A), Di- n- butyladipat (Cetiol® B), Myristylmyristat (Cetiol® MM), Cetearyl Isononanoate (Cetiol® SN), Ölsäuredecylester (Cetiol® V).
- Dicarbonsäureester wie Di- n- butyladipat, Di-(2- ethylhexyl)-adipat, Di-(2- ethylhexyl)-succinat und Di- isotridecylacelaat sowie Diolester wie Ethylenglykol- dioleat, Ethylenglykol- di- isotridecanoat, Propylenglykol- di (2- ethylhexanoat), Propylenglykol- diisostearat, Propylenglykol- di- pelargonat, Butandiol- di- isostearat, Neopentylglykoldicaprylat,
- symmetrische, unsymmetrische oder cyclische Ester der Kohlensäure mit Fettalkoholen, beispielsweise beschrieben
   in der DE-OS 197 56 454, Glycerincarbonat oder Dicaprylylcarbonat (Cetiol® CC),
- Trifettsäureester von gesättigten und/ oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I), 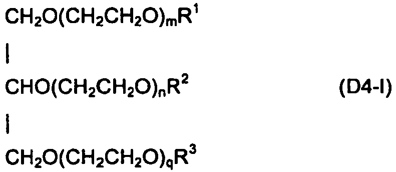 in der R¹, R² und R³ unabhängig voneinander für Wasserstoff oder für einen linearen oder verzweigten, gesättigten und/ oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R1 für einen Acylrest und R2 und R3 für Wasserstoff und die Summe (m+n+q) ist 0. Typische Beispiele sind Mono- und/oder Diglyceride auf Basis von Capronsäure, Caprylsäure, 2- Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den Zusammensetzungen für das erfindungsgemäße kosmetische Verfahren gemäß Anspruch 1 und die erfindungsgemäße Verwendung gemäß Anspruch 12 beträgt üblicherweise 0,1 bis 30 Gew.%, bezogen auf die gesamte Zusammensetzung, bevorzugt 0,1 bis 20 Gew.%, und insbesondere 0,1 bis 15 Gew.%.

Die Gesamtmenge an Öl- und Fettkomponenten in den Zusammensetzungen für das erfindungsgemäße kosmetische Verfahren gemäß Anspruch 1 und die erfindungsgemäße Verwendung gemäß Anspruch 12 beträgt üblicherweise 0,1 - 75 Gew.%, bezogen aus die gesamte Zusammensetzung. Mengen von 0,1 - 35 Gew.% sind erfindungsgemäß bevorzugt. Weitere zusätzliche Bestandteile zum erfindungsgemäßen Wirkstoff (A) können in den Mitteln oberflächenaktive Verbindungen enthalten sein, insbesondere solche aus der Gruppe der anionischen, amphoteren, zwitterionischen und/oder nichtionischen Tenside (E).

Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen

Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wäßriger Lösung stark hydratisiert sind. Weitergehende Definitionen und Eigenschaften von Tensiden finden sich in "H.-D.Dörfler, Grenzflächen- und Kolloidchemie, VCH Verlagsgesellschaft mbH. Weinheim, 1994". Die zuvor wiedergegebene Begriffsbestimmung findet sich ab S. 190 in dieser Druckschrift.

Als anionische Tenside (E1) eignen sich in den erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z.B. eine Carboxylat-, Sulfat-, Sulfonat- oder Phosphat- Gruppe und eine lipophile Alkylgruppe mit etwa 8 bis 30 C- Atomen. Zusätzlich können im Molekül Glykol- oder Polyglykolether- Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C- Atomen in der Alkanolgruppe,
- lineare und verzweigte Fettsäuren mit 8 bis 30 C- Atomen (Seifen),
- Ethercarbonsäuren der Formel R- O-(CH2-CH20)x CH2-COOH, in der R eine lineare Alkylgruppe mit 8 bis 30 C- Atomen und x = 0 oder 1 bis 16 ist,
- Acylsarcoside mit 8 bis 24 C- Atomen in der Acylgruppe,
- Acyltauride mit 8 bis 24 C- Atomen in der Acylgruppe,
- Acylisethionate mit 8 bis 24 C- Atomen in der Acylgruppe,
- Sulfobernsteinsäuremono- und -dialkylester mit 8 bis 24 C- Atomen in der Alkylgruppe und Sulfobernsteinsäure monoalkyl polyoxyethylester mit 8 bis 24 C- Atomen in der Alkylgruppe und 1 bis 6 Oxyethylgruppen,
- lineare Alkansulfonate mit 8 bis 24 C- Atomen,
- lineare Alpha- Olefinsulfonate mit 8 bis 24 C- Atomen,
- Alpha- Sulfofettsäuremethylester von Fettsäuren mit 8 bis 30 C- Atomen,
- Alkylsulfate und Alkylpolyglykolethersulfate der Formel R-O-(CH2-CH2O)x-OSO3H, in der R eine bevorzugt lineare Alkylgruppe mit 8 bis 30 C- Atomen und x = 0 oder 1 bis 12 ist,
- Gemische oberflächenaktiver Hydroxysulfonate gemäß DE-A-37 25 030,
- sulfatierte Hydroxyalkylpolyethylen- und/ oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 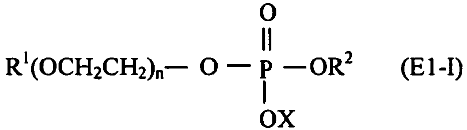 in der R1 bevorzugt für einen aliphatischen Kohlenwasserstoffrest mit 8 bis 30 Kohlenstoffatomen, R2 für Wasserstoff, einen Rest (CH2CH2O)nR1 oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4-Kohlenwasserstoffrest, steht,
- sulfatierte Fettsäurealkylenglykolester der Formel (E1 -II)

   R⁷CO(AkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (E1-III) 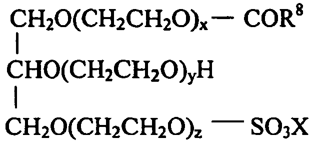 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid (ether) sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R8CO für einen linearen Acylrest mit 8 bis 18 Kohlenstoffatomen steht, wie sie beispielsweise in der EP-B1 0 561 825, der EP-B1 1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind,
- Amidethercarbonsäuren wie sie in der EP 0 690 044 beschrieben sind,
- Kondensationsprodukte aus C8-C30 - Fettalkoholen mit Proteinhydrolysaten und/oder Aminosäuren und deren Derivaten, welche dem Fachmann als Eiweissfettsäurekondensate bekannt sind, wie beispielsweise die Lamepon® -Typen, Gluadin® - Typen, Hostapon® KCG oder die Amisoft® - Typen.
Bevorzugte anionische Tenside sind Alkylsulfate, Alkylpolyglykolethersulfate und Ethercarbonsäuren mit 10 bis 18 C- Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis
18 C- Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine COO⁻ - oder -SO₃⁻ - Gruppe tragen. Besonders geeignete zwitterionische Tenside sind die sogenannten Betaine wie die N- Alkyl- N, N- dimethylammonium-glycinate, beispielsweise das Kokosalkyldimethylammoniumglycinat, N-Acyl-aminopropyl-N, N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer C8-C24 - Alkyl- oder -Acylgruppe im Molekül mindestens eine freie Aminogruppe und mindestens eine -COOH oder -SO₃H- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C12-C18 - Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/ oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C2-C6 - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- C12-C30-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)
R¹CO-(OCH₂CHR²)_{w}OR³ (E4-I)

in der R¹CO für einen linearen oder verzweigten, gesättigten und/ oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFWJoumal Heft 8, 598 (1995) verwiesen.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/ oder Alkenyloligoglucoside. Die Indexzahl p in der allgemeinen Formel (E4-II) gibt den Oligomerisierungsgrad (DP), d. h. die Verteilung von Mono-und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/ oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R4 kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C8-C10 (DP = 1 bis 3), die als Vorlauf bei der destillativen Auftrennung von technischem C8-C18-Kokosfettalkohol anfallen und mit einem Anteil von weniger als 6 Gew.-% C12-Alkohol verunreinigt sein können sowie Alkyloligoglucoside auf Basis technischer C9/11-Oxoalkohole (DP = 1 bis 3). Der Alkyl- bzw. Alkenylrest R15 kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C12/14-Kokosalkohol mit einem DP von 1 bis 3.

Zuckertenside vom Typ der Fettsäure- N- alkylpolyhydroxyalkylamide, ein nichtionisches Tensid der Formel (E4-III), 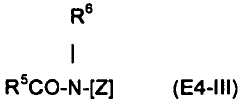 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkykest mit 3 bis 12 Kohlenstoffatomen und 3 bis 10 Hydroxylgruppen steht. Bei den Fettsäure- N- alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US Patentschriften US 1,985,424, US 2,016,962 und US 2,703,798 sowie die Internationale Patentanmeldung WO 92/06984 verwiesen. Eine Übersicht zu diesem Thema von H.Kelkenberg findet sich in Tens. Surf. Det. 25, 8 (1988). Vorzugsweise leiten sich die Fettsäure- N- alkylpolyhydroxyalkylamide von reduzierenden Zuckern mit 5 oder 6 Kohlenstoffatomen, insbesondere von der Glucose ab. Die bevorzugten Fettsäure- N-alkylpolyhydroxyalkylamide stellen daher Fettsäure N- alkylglucamide dar, wie sie durch die Formel (E4- IV) wiedergegeben werden:

R⁷CONR⁸-CH₂-(CHOH)₄-CH₂OH (E4-IV)

Vorzugsweise werden als Fettsäure- N- alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure- N- alkylglucamide der Formel (E4- IV), die durch reduktive Aminierung von Glucose mit Methylamin und anschließende Acylierung mit Laurinsäure oder C12/14 Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid- Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2- Stellung methylverzweigte aliphatische Reste. Solche Alkylreste sind beispielsweise 1- Octyl, 1- Decyl, 1- Lauryl, 1- Myristyl, 1- Cetyl und 1- Stearyl. Besonders bevorzugt sind 1- Octyl, 1- Decyl, 1- Lauryl, 1- Myristyl. Bei Verwendung sogenannter "OxoAlkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den Zusammensetzungen für das erfindungsgemäße kosmetische Verfahren gemäß Anspruch 1 und die erfindungsgemäße Verwendung gemäß Anspruch 12 bevorzugt in Mengen von 0,1 - 20 Gew.%, bezogen auf die gesamte Zusammensetzung, enthalten sein. Mengen von 0,5 - 15 Gew.- % sind bevorzugt, und ganz besonders bevorzugt sind Mengen von 0,5 - 7,5 Gew. %.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/ oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden
dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Neben den genannten Komponenten können die Mittel als oberflächenaktive Verbindungen kationische Tenside (E5) vom Typ der quarternären Ammoniumverbindungen, der Esterquats und der Amidoamine enthalten. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI- Bezeichnungen Quaternium- 27 und Quaternium- 83 bekannten tmidazolium- Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquais sind quaternierte Estersalze von Fettsäuren mit Triethanolamin, quatemierte Estersalze von Fettsäuren mit Diethanolalkylaminen und quaternierten Estersalzen von Fettsäuren mit 1,2- Dihydroxypropyldialkylaminen. Solche Produkte werden beispielsweise unter den Warenzeichen Stepantex® , Dehyquart® und Armocare® vertrieben. Die Produkte Armocare® VGH- 70, ein N, N-Bis (2- Palmitoyloxyethyl) dimethylammoniumchlorid, sowie Dehyquart® F- 75, Dehyquart® C- 4046, Dehyquart® L80 und Dehyquart® AU- 35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Die kationischen Tenside (E5) sind in den Zusammensetzungen für das erfindungsgemäße kosmetische Verfahren gemäß Anspruch 1 und die erfindungsgemäße Verwendung gemäß Anspruch 12 bevorzugt in Mengen von 0,05 bis 10 Gew.%, bezogen auf die gesamte Zusammensetzung, enthalten. Mengen von 0,1 bis 5 Gew.% sind besonders bevorzugt.

Die Tenside (E) werden in Mengen von 0,1 - 50 Gew.%, bevorzugt 0,5 - 30 Gew.% und ganz besonders bevorzugt von 0,5 - 25 Gew.%, bezogen auf die gesamte Zusammensetzung für das erfindungsgemäße kosmetische Verfahren gemäß Anspruch 1 und die erfindungsgemäße Verwendung gemäß Anspruch 12, eingesetzt.

In einer weiteren bevorzugten Ausführungsform werden in den Zusammensetzungen für das erfindungsgemäße kosmetische Verfahren gemäß Anspruch 1 und die erfindungsgemäße Verwendung gemäß Anspruch 12 Emulgatoren (F) verwendet. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D.Dörfler, Grenzflächen- und Kolloidchemie, VCH Verlagsgesellschaft mbH. Weinheim, 1994". Erfindungsgemäß verwendbare Emulgatoren sind beispielsweise
- Anlagerungsprodukte von 4 bis 30 Mol Ethylenoxid und/ oder 0 bis 5 Mol Propylenoxid an lineare Fettalkohole mit 8 bis 22 C- Atomen, an Fettsäuren mit 12 bis 22 C- Atomen und an Alkylphenole mit 8 bis 15 C- Atomen in der Alkylgruppe,
- C12-C22-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin- Anlagerungsprodukte an Methylglucosid- Fettsäureester, Fettsäurealkanolamide und Fettsäureglucamide,
- C8-C22-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov® 68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C- Atomen,
- Sterine. Als Sterine wird eine Gruppe von Steroiden verstanden, die am C- Atom 3 des Steroid- Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose- Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly- 12-hydroxystearat (Handelsprodukt Dehymuls ® PGPH),
- Lineare und verzweigte Fettsäuren mit 8 bis 30 C - Atomen und deren Na-, K-, Ammonium-, Ca-, Mg- und Zn - Salze.

Die Zusammensetzungen für das erfindungsgemäße kosmetische Verfahren gemäß Anspruch 1 und die erfindungsgemäße Verwendung gemäß Anspruch 12 enthalten die Emulgatoren bevorzugt in Mengen von 0,1 - 25 Gew.%, insbesondere 0,1 - 15 Gew.%, bezogen auf die gesamte Zusammensetzung.

Bevorzugt können die Zusammensetzungen für das erfindungsgemäße kosmetische Verfahren gemäß Anspruch 1 und die erfindungsgemäße Verwendung gemäß Anspruch 12 mindestens einen nichtionogenen Emulgator mit einem HLB- Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten Nicht-ionogene Emulgatoren mit einem HLB- Wert von 10 -15 können erfindungsgemäß besonders bevorzugt sein.

Als weiterhin vorteilhaft hat es sich gezeigt, daß Polymere (G) die Wirkung des erfindungsgemäßen Wirkstoffes (A) unterstützen können. **In einer bevorzugten** Ausführungsform werden den Zusammensetzungen für das erfindungsgemäße kosmetische Verfahren gemäß Anspruch 1 und die erfindungsgemäße Verwendung gemäß Anspruch 12 daher Polymere zugesetzt, wobei sich sowohl kationische, anionische, amphotere als auch nichtionische Polymere als wirksam erwiesen haben.

Unter kationischen Polymeren (G1) sind Polymere zu verstehen, welche in der Haupt- und/ oder Seitenkette eine Gruppe aufweisen, welche "temporär" oder "permanent" kationisch sein kann. Als "permanent kationisch" werden erfindungsgemäß solche Polymere bezeichnet, die unabhängig vom pH- Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4 Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 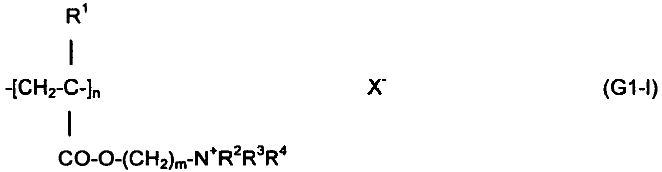 in der R¹= -H oder -CH₃ ist, R², R³ und R⁴ unabhängig voneinander ausgewählt sind aus C1-4-Alkyl-, Alkenyl- oder Hydroxyalkylgruppen, m = 1, 2, 3 oder 4, n eine natürliche Zahl und X- ein physiologisch verträgliches organisches oder anorganisches Anion ist, sowie Copolymere, bestehend im wesentlichen aus den in Formel (G1- I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches Gegenionen X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly (methacryloyloxyethyltrimethylammoniumchlorid) mit der INCI- Bezeichnung Polyquaternium- 37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew. % aufweisen sollte, eingesetzt. Solche Polymer-dispersionen sind unter den Bezeichnungen Salcare® SC 95 (ca. 50 % Polymeranteil, weitere Komponenten: Mineralöl (INCI- Bezeichnung: Mineral Oil) und Tridecyl-polyoxypropylenpolyoxyethylen- ether (INCI- Bezeichnung: PPG-1-Trideceth- 6)) und Salcare® SC 96 (ca. 50 % Polymeranteil, weitere Komponenten: Mischung von Diestern des Propylenglykols mit einer Mischung aus Capryl- und Caprinsäure (INCI- Bezeichnung: Propylene Glycol Dicaprylate/ Dicaprate) und Tridecyl- polyoxypropylen- polyoxyethylen- ether (INCIBezeichnung: PPG-1-Trideceth- 6)) im Handel erhältlich.

Copolymere mit Monomereinheiten gemäß Formel (G1- I) enthalten als nichtionogene Monomereinheiten bevorzugt Acrylamid, Methacrylamid, Acrylsäure- C1-4-alkylester und Methacrylsäure- C1-4-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid- Copolymer. Solche Copolymere, bei denen die Monomere in einem Gewichtsverhältnis von etwa 20: 80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise
- quaternisierte Cellulose- Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quatemierte Cellulose- Derivate,
- kationische Alkylpolyglycoside gemäß der DE- PS 44 13 686,
- kationiserter Honig, beispielsweise das Handelsprodukt Honeyquat® 50,
- kationische Guar- Derivate, wie insbesondere die unter den Handelsnamen Cosmedia® Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylamino- modifiziertes Silicon, das auch als Amodimethicone bezeichnet wird), SM-2059 (Hersteller: General Electric), SLM- 55067 (Hersteller: Wacker) sowie Abil® -Quat 3270 und 3272 (Hersteller: Th. Goldschmidt), diquaternäre Polydimethylsiloxane, Quatemium- 80),
- polymere Dimethyldiallylammoniumsalze und deren Copolymere mit Estern und Amiden von Acrylsäure und Methacrylsäure. Die unter den Bezeichnungen Merquat® 100 (Poly (dimethyldiallylammoniumchlorid)) und Merquat® 550 (Dimethyldiallylammoniumchlorid-Acrylamid- Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon- Dimethylaminoethylmethacrylat- Copolymere. Solche Verbindungen sind unter den Bezeichnungen Gafquat® 734 und Gafquat® 755 im Handel erhältlich,
- Vinylpyrrolidon- Vinylimidazoliummethochlorid- Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen Polyquaternium 2, Polyquaternium 17, Polyquatemium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquatemium- 24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat® ASCP 1011, Gafquat® HS 110, Luviquat® 8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/ 101 im Handel frei verfügbar sind.

Erfindungsgemäß bevorzugte kationische Polymere sind kationische Cellulose- Derivate und Chitosan und dessen Derivate, insbesondere die Handelsprodukte Polymer® JR 400, Hydagen® HCMF und Kytamer® PC, kationische Guar- Derivate, kationische Honig- Derivate, insbesondere das Handelsprodukt Honeyquat® 50, kationische Alkylpolyglycodside gemäß der DE- PS 44 13 686 und Polymere vom Typ Polyquaternium-37.

Bei den anionischen Polymeren (G2), welche mit den Wirkstoffen (A) in den erfindungsgemäßen Mitteln verwendet werden können, handelt es sich um anionische Polymere, welche Carboxylat- und/ oder Sulfonatgruppen aufweisen. Beispiele für anionische Monomere, aus denen derartige Polymere bestehen können, sind Acrylsäure, Methacrylsäure,
Crotonsäure, Maleinsäureanhydrid und 2- Acrylamido-2-methylpropansulfonsäure. Dabei können die sauren Gruppen ganz oder teilweise als Natrium-, Kalium-, Ammonium-, Mono- oder Triethanolammonium- Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co- Monomer 2- Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Beispielsweise ist ein solches Homopolymer der 2-Acrylamido-2-methylpropan-sulfonsäure, unter der Bezeichnung Rheothik® 11-80 im Handel erhältlich.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure- Acrylamid- Copolymere sowie insbesondere Polyacrylamidcopolymere mit Sulfonsäuregruppen- haltigen Monomeren. Ein besonders bevorzugtes anionisches Copolymer besteht aus 70 bis 55 Mol- % Acrylamid und 30 bis 45 Mol-% 2- Acrylamido-2-methylpropansulfonsäure, wobei die Sulfonsäuregruppe
ganz oder teilweise als Natrium-, Kalium-, Ammonium-, Mono- oder Triethanolammonium- Salz vorliegt. Dieses Copolymer kann auch vernetzt vorliegen, wobei als Vemetzungsagentien bevorzugt polyolefinisch ungesättigte Verbindungen wie Tetraallyloxyethan, Allylsucrose, Allylpentaerythrit und Methylen-bisacrylamid zum Einsatz kommen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls gut geeignete Polymere. Ein mit 1,9- Decadiene vernetztes Maleinsäure- Methylvinylether- Copolymer ist unter der Bezeichnungg Stabileze® QM im Handel erhältlich.

Weiterhin können als Polymere in allen Zusammensetzungen für das erfindungsgemäße kosmetische Verfahren gemäß Anspruch 1 und die erfindungsgemäße Verwendung gemäß Anspruch 12 amphotere Polymere (G3) verwendet werden. Unter dem Begriff amphotere Polymere werden sowohl solche Polymere, die im Molekül sowohl freie Aminogruppen als auch freie -COOH- oder SO₃H- Gruppen enthalten und zur Ausbildung innerer Salze befähigt sind, als auch zwitterionische Polymere, die im Molekül quartäre Ammoniumgruppen und -COO⁻-oder - SO₃⁻Gruppen enthalten, und solche Polymere zusammengefaßt, die -COOH- oder SO₃H Gruppen und quartäre Ammoniumgruppen enthalten.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N⁽⁺⁾R³R⁴R⁵A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH- Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3- II),

   R⁶-CH=CR⁷-COOH (G3-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH- Gruppe und A⁽⁻⁾ ein Halogenid-, Methoxysulfat- oder Ethoxysulfat-Ion ist; Acrylamidopropyl- trimethylammoniumchlorid ist ein besonders bevorzugtes Monomeres (a). Als Monomeres (b) für die genannten Polymerisate wird bevorzugt Acrylsäure verwendet.

Weiterhin können in allen Zusammensetzungen für das erfindungsgemäße kosmetische Verfahren gemäß Anspruch 1 und die erfindungsgemäße Verwendung gemäß Anspruch 12 nichtionogene Polymere (G4) enthalten sein.

Geeignete nichtionogene Polymere sind beispielsweise:
- Vinylpyrrolidon/ Vinylester- Copolymere, wie sie beispielsweise unter dem Warenzeichen Luviskol® (BASF) vertrieben werden. Luviskol® VA 64 und Luviskol® VA 73, jeweils Vinylpyrrolidon/ Vinylacetat- Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden.
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/ oder Hydroxy- Gruppen enthalten.
- Glycosidisch substituierte Silicone gemäß der EP 0612759 B1.

Es ist erfindungsgemäß auch möglich, dass in den Zusammensetzungen für das erfindungsgemäße kosmetische Verfahren gemäß Anspruch 1 und die erfindungsgemäße Verwendung gemäß Anspruch 12 mehrere, insbesondere zwei verschiedene Polymere gleicher Ladung und/oder jeweils ein ionisches und ein amphoteres und/oder nicht ionisches Polymer enthalten sind.

Die Polymere (G) sind in den Zusammensetzungen für das erfindungsgemäße kosmetische Verfahren gemäß Anspruch 1 und die erfindungsgemäße Verwendung gemäß Anspruch 12 bevorzugt in Mengen von 0,01 bis 10 Gew.%, bezogen auf die gesamte Zusammensetzung, enthalten. Mengen von 0,1 bis 5, insbesondere von 0,1 bis 3 Gew.- %, sind besonders bevorzugt. Weiterhin können in den Zusammensetzungen für das erfindungsgemäße kosmetische Verfahren gemäß Anspruch 1 und die erfindungsgemäße Verwendung gemäß Anspruch 12 Proteinhydrolysate und/oder Aminosäuren und deren Derivate (H) enthalten sein. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind LAlanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß- Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita- Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure- Kondensationsprodukte. Solche Produkte werden beispielsweise unter den Bezeichnungen Lamepon® (Cognis), Lexein® (Inolex), Crolastin® (Croda) oder Crotein® (Croda) vertrieben.

Die Proteinhydrolysate oder deren Derivate sind in den Zusammensetzungen für das erfindungsgemäße kosmetische Verfahren gemäß Anspruch 1 und die erfindungsgemäße Verwendung gemäß Anspruch 12 bevorzugt in Mengen von 0,1 bis 10 Gew.%, bezogen auf die gesamte Zusammensetzung, enthalten. Mengen von 0,1 bis 5 Gew.% sind besonders bevorzugt. Weiterhin können 2- Pyrrolidinon-5-carbonsäure und/ oder deren Derivate (J) in den Zubereitungen des erfindungsgemäßen Verfahrens verwendet werden. Bevorzugt sind die Natrium-, Kalium-, Calcium-, Magnesium- oder Ammoniumsalze, bei denen das Ammoniumion neben Wasserstoff eine bis drei C1- bis C4-Alkylgruppen trägt. Das Natriumsalz ist ganz besonders bevorzugt. Die eingesetzten Mengen in den erfindungsgemäßen Mitteln betragen 0,01 bis 10 Gew. %, bezogen auf das gesamte Mittel, besonders bevorzugt 0,1 bis 5, und insbesondere 0,1 bis 3 Gew. %.

Ebenfalls als vorteilhaft hat sich die Verwendung von Vitaminen, Provitaminen und Vitaminvorstufen sowie deren Derivaten (K) erwiesen.

Dabei sind erfindungsgemäß solche Vitamine, Pro- Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A1) sowie das 3,4-Didehydroretinol (Vitamin A2). Das ß-Carotin ist das Provitamin des Retinols. Als Vitamin A- Komponente kommen erfindungsgemäß beispielsweise Vitamin A- Säure und deren Ester, Vitamin A- Aldehyd und Vitamin A- Alkohol sowie dessen Ester wie das Palmitat und das Acetat in Betracht Die erfindungsgemäß verwendeten Zubereitungen enthalten die Vitamin A-Komponente bevorzugt in Mengen von 0,05-1 Gew.- %, bezogen auf die gesamte Zubereitung.

Zur Vitamin B- Gruppe oder zu dem Vitamin B- Komplex gehören u. a.
- Vitamin B1 (Thiamin)
- Vitamin B2 (Riboflavin)
- Vitamin B3. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.- %, bezogen auf das gesamte Mittel, enthalten ist.
- Vitamin B5 (Pantothensäure, Panthenol und Pantolacton). Im Rahmen dieser Gruppe wird bevorzugt das Panthenol und/ oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B5-Typs sind in den erfindungsgemäß verwendeten Mitteln bevorzugt in Mengen von 0,05 - 10 Gew.- %, bezogen auf das gesamte Mittel, enthalten. Mengen von 0,1- 5 Gew.- % sind besonders bevorzugt.
- Vitamin B6 (Pyridoxin sowie Pyridoxamin und Pyridoxal).

Vitamin C (Ascorbinsäure). Vitamin C wird in den erfindungsgemäß verwendeten Mitteln bevorzugt in Mengen von 0,1 bis 3 Gew.- %, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den Zusammensetzungen für das erfindungsgemäße kosmetische Verfahren gemäß Anspruch 1 und die erfindungsgemäße Verwendung gemäß Anspruch 12 bevorzugt in Mengen von 0,05-1 Gew.%, bezogen auf die gesamte Zusammensetzung, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS, 4S, 6aR)-2-Oxohexahydrothienol [3,4-d]-imidazol-4-valeriansäure bezeichnet, für die sich aber inzwischen der Trivialname Biotin durchgesetzt hat. Biotin ist in den Zusammensetzungen für das erfindungsgemäße kosmetische Verfahren gemäß Anspruch 1 und die erfindungsgemäße Verwendung gemäß Anspruch 12 bevorzugt in Mengen von 0,0001 bis 1,0 Gew.%, insbesondere in Mengen von 0,001 bis 0,01 Gew.% enthalten.

Bevorzugt enthalten die erfindungsgemäß verwendeten Zubereitungen Vitamine, Provitamine und Vitaminvorstufen aus den Gruppen A, B, E und H. Selbstverständlich können auch mehrere Vitamine und Vitaminvorstufen gleichzeitig enthalten sein.

Panthenol, Pantolacton, Pyridoxin und seine Derivate sowie Nicotinsäureamid und Biotin sind besonders bevorzugt. Die Einsatzmenge an Vitaminen und Vitaminvorstufen in den Zusammensetzungen für das erfindungsgemäße kosmetische Verfahren gemäß Anspruch 1 und die erfindungsgemäße Verwendung gemäß Anspruch 12 beträgt üblicherweise 0,0001 - 10 Gew.%, bezogen auf die gesamte Zusammensetzung, bevorzugt 0,0001 - 5 Gew.%, und insbesondere 0,0001 - 3 Gew.%.

Schließlich können in den Zusammensetzungen für das erfindungsgemäße kosmetische Verfahren gemäß Anspruch 1 und die erfindungsgemäße Verwendung gemäß Anspruch 12 Pflanzenextrakte (L) verwendet werden.

Üblicherweise werden diese Extrakte durch Extraktion der gesamten Pflanze hergestellt. Es kann aber in einzelnen Fällen auch bevorzugt sein, die Extrakte ausschließlich aus Blüten und/ oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdom, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Cluendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Cluendel, Schafgarbe, Hauhechel, Meristem, Ginseng und Ingwerwurzel. Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/ Propylenglykol im Verhältnis 1: 10 bis 10: 1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.- % Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Die Einsatzmenge der Pflanzenextrakte in den Zusammensetzungen für das erfindungsgemäße kosmetische Verfahren gemäß Anspruch 1 und die erfindungsgemäße Verwendung gemäß Anspruch 12 beträgt üblicherweise 0,01 - 50 Gew.%, bezogen auf die gesamte Zusammensetzung, bevorzugt 0,1 - 30 Gew.%, und insbesondere 0,1 - 20 Gew.%.

Vorteilhaft im Sinne der Erfindung können zusätzlich kurzkettige Carbonsäuren (N) eingesetzt werden. Ganz besonders bevorzugt kann es erfindungsgemäß sein, diese kurzkettigen Carbonsäuren mit den erfindungsgemäßen Wirkstoffen der Formel (A-I) in Form der aus diesen beiden Komponenten gebildeten Mischsalze zu verwenden. Unter kurzkettigen Carbonsäuren und deren Derivaten im Sinne der Erfindung werden Carbonsäuren verstanden, welche gesättigt oder ungesättigt und/ oder geradkettig oder verzweigt oder cyclisch und/ oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C- Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphonoder Phosphatester vorliegen. Die erfindungsgemäßen Carbonsäuren können selbstverständlich entlang der Kohlenstoffkette oder des Ringgerüstes substituiert sein. Zu den Substituenten der erfindungsgemäßen Carbonsäuren sind beispielsweise zu zählen C1- C8- Alkyl-, C2- C8- Alkenyl-, Aryl-, Aralkyl- und Aralkenyl-, Hydroxymethyl-, C2- C8- Hydroxyalkyl-, C2- C8- Hydroxyalkenyl-, Aminomethyl-, C2- C8- Aminoalkyl-, Cyano-, Formyl-, Oxo-, Thioxo-, Hydroxy-, Mercapto-, Amino-, Carboxy- oder Iminogruppen. Bevorzugte Substituenten sind C1- C8- Alkyl-, Hydroxymethyl-, Hydroxy-, Amino- und Carboxygruppen. Besonders bevorzugt sind Substituenten in α - Stellung. Ganz besonders bevorzugte Substituenten sind Hydroxy-, Alkoxy- und Aminogruppen, wobei die Aminofunktion gegebenenfalls durch Alkyl-, Aryl-, Aralkylund/oder Alkenylreste weiter substituiert sein kann. Weiterhin sind ebenfalls bevorzugte Carbonsäurederivate die Phosphon- und Phosphatester.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o, m, p- Phthalsäure, Naphthoesäure, - Toluoylsäure, Hydratropasäure, Atropasäure, Zimtsäure, Isonicotinsäure, Nicotinsäure, Bicarbaminsäure, 4,4'- Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 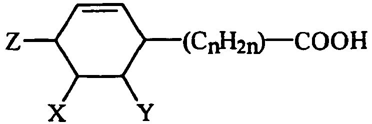 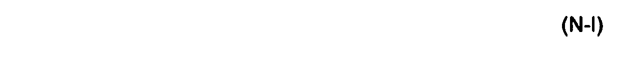 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH- Gruppe und die andere für Wasserstoff oder einen Methyl- oder Ethylrest, Dicarbonsäuren der allgemeinen Formel (N- I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt.

Ein Herstellungsverfahren ist beispielsweise der US- Patentschrift 3,753,968 zu entnehmen. Die Dicarbonsäuren der Formel (N-I) können beispielsweise durch Umsetzung von mehrfach ungesättigten Dicarbonsäuren mit ungesättigten Monocarbonsäuren in Form einer Diels- Alder-Cyclisierung hergestellt werden. Üblicherweise wird man von einer mehrfach ungesättigten Fettsäure als Dicarbonsäurekomponente ausgehen. Bevorzugt ist die aus
natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels- Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl- Substituenten am Cyclohexylring unterscheiden oder aus diesen Verbindungen formal durch Anlagerung von einem Molekül Wasser an die Doppelbildung des Cyclohexenrings gebildet werden. Als erfindungsgemäß besonders vorteilhaft hat sich die Dicarbonsäure (- mischung) erwiesen, die durch Umsetzung von Linolsäure mit Acrylsäure entsteht Es handelt sich dabei um eine Mischung aus 5- und 6- Carboxy- 4- hexyl- 2- cyclohexen-1- octansäure. Solche Verbindungen sind kommerziell unter den Bezeichnungen Westvaco Diacid® 1550 und Westvaco Diacid® 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl- Ammoniumsalze zu verstehen sind. Ganz besonders bevorzugt können im Rahmen der Erfindung jedoch mit alkalisch reagierenden Aminosäuren, wie beispielsweise Arginin, Lysin, Omithin und Histidin, neutralisierte Säuren eingesetzt werden. Weiterhin kann es aus Fonnulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Ganz besonders bevorzugt kann es erfindungsgemäß sein, unter den kurzkettigen Carbonsäuren insbesondere die Hydroxycabonsäuren zur Mischsalzbildung mit den erfindungsgemäßen Wirkstoffen der Formel (A- I) zu verwenden. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeignete Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8-22 C- Atomen, also z.B. Fettalkohole oder synthetische Fettalkohole. Dabei sind die Ester von C12- C15- Feftalkoholen besonders bevorzugt. Ester dieses Typs sind im Handel erhältlich, z.B. unter dem Warenzeichen Cosmacol® der EniChem, Augusta Industriale. Besonders bevorzugte Polyhydroxypolycarbonsäuren sind Polymilchsäure und Polyweinsäure sowie deren Ester.

Als weitere Inhaltsstoffe, welche die Wirkung des Wirkstoffes der Formel (A- I) fördern, können heterocyclische Verbindungen wie beispielsweise Imidazol, Pyrrolidin, Piperidin, Dioxolan, Dioxan, Morpholin und Piperazin eingesetzt werden. Weiterhin eignen sich Derivate dieser Verbindungen wie beispielsweise die C1-4-Alkyl- Derivate, C1-4-Hydroxyatkyl- Derivate und C1-4-Aminoalkyl- Derivate. Bevorzugte Substituenten, die
sowohl an Kohlenstoffatomen als auch an Stickstoffatomen der heterocyclischen Ringsysteme positioniert sein können, sind Methyl-, Ethyl-, β-Hydroxyethyl- und β-Aminoethyl- Gruppen. Bevorzugt enthalten diese Derivate 1 oder 2 dieser Substituenten.

Erfindungsgemäß bevorzugte Derivate heterocyclischer Verbindungen sind beispielsweise 1-Methylimidazol, 2-Methylimidazol, 4(5)-Methylimidazol, 1,2-Dimethylimidazol, 2-Ethylimidazol, 2-Isopropylimidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin. Weiterhin erfindungsgemäß bevorzugte Imidazolderivate sind Biotin, Hydantoin und Benzimidazol.

Unter diesen heterocyclischen Pflegestoffen sind die Mono- und Dialkylimidazole, Biotin, Hydantoin, sowie insbesondere das Imidazol selbst besonders bevorzugt.

Diese heterocyclischen Verbindungen sind in den Zusammensetzungen für das erfindungsgemäße kosmetische Verfahren gemäß Anspruch 1 und die erfindungsgemäße Verwendung gemäß Anspruch 12 in Mengen von 0,5 bis 10 Gew.%, bezogen auf die gesamte Zusammensetzung, enthalten. Mengen von 2 bis 6 Gew.- % haben sich als besonders geeignet erwiesen.

Zusätzlich kann es sich als vorteilhaft erweisen, wenn die Zusammensetzungen für das erfindungsgemäße kosmetische Verfahren gemäß Anspruch 1 und die erfindungsgemäße Verwendung gemäß Anspruch 12 Penetrationshilfsstoffe und/oder Quellmittel (M) enthalten. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2- Diole und 1,3- Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Die Penetrationshilfsstoffe und Quellmittel sind in den erfindungsgemäß eingesetzten Zubereitungen in Mengen von 0,1 bis 20 Gewichts- %, bezogen auf das gesamte Mittel, enthalten. Mengen von 01, bis 10 Gewichts- % sind bevorzugt.

Weiterhin sind als konditionierende Wirkstoffe geeignet Silikonöle und Silikon- Gums, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie das Handelsprodukt Fancorsil® LIM-1.

Erfindungsgemäß als konditionierende Wirkstoffe ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning® 939 Emulsion (enthaltend ein hydroxylamino- modifiziertes Silicon, das auch als Amodimethicone bezeichnet wird), SM- 2059 (Hersteller: General Electric), SLM- 55067 (Hersteller: Wacker) sowie Abil® -Quat 3270 und 3272 (Hersteller: Th. Goldschmidt; diquaternäre Polydimethylsiloxane, Quatemium- 80). Ein geeignetes anionisches Silikonöl ist das Produkt Dow Corning® 1784.

Weitere Wirk-, Hilfs- und Zusatzstoffe sind beispielsweise
- Verdickungsmittel wie Agar- Agar, Guar- Gum, Alginate, Xanthan- Gum, Gummi arabicum, Karaya- Gummi, Johannisbrotkernmehl, Leinsamengummen, Dextrane, Cellulose- Derivate, z. B. Methylcellulose, Hydroxyalkylcellulose und Carboxymethylcellulose, Stärke- Fraktionen und Derivate wie Amylose, Amylopektin und Dextrine, Tone wie z.B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkemöl sowie
- quatemierte Amine wie Methyl-1-alkylamidoethyl-2-alkylimidazolinium- methosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/ PVP- und Styrol/ Acrylamid- Copolymere
- Perlglanzmittel wie Ethylenglykolmono- und -distearat sowie PEG-3-distearat,
- Pigmente,
- Treibmittel wie Propan- Butan- Gemische, N2O, Dimethylether, CO2 und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die Monographie von K. H. Schrader, Grundlagen und Rezepturen der Kosmetika, 2. Auflage, Hüthig Buch Verlag Heidelberg, 1989, verwiesen.

Die folgenden Beispiele sollen die Erfindung näher erläutern.

### Beispiele

Soweit nicht anders vermerkt, sind alle Angaben Gewichtsteile

### 1. Wirkungsnachweis

Zur Untersuchung der Wirkung der erfindungsgemäßen Wirkstoffe (A) wurde das in vitro Modell der humanen Haarfollikel von Philpott et al., veröffentlicht in Basic Cell Culture Protocols, Humana Press, Totowa, New Jersey, 1997, herangezogen. Auf diese Publikation wird ausdrücklich Bezug genommen. Gemäß dieser Veröffentlichung wurde wie folgt verfahren:

### a. Isolation humaner Kopfhauthaarfollikel

Es wird okzipitale, humane Vollhaut isoliert.

### b. Aufreinigung der isolierten humanen Kopfhautfollikel

Die herausgezupften Haarfollikel werden in ein frisches Isolationsmedium überführt, darin gesammelt und anschließend mehrfach unter sterilen Bedingungen mit frischem Medium gewaschen.

Je drei Haarfollikel werden randomisiert in ein Well einer 24-Well Platte mit 500µl kompletten Mediums (verwendet wird Williams E Medium, supplementiert mit 2 mM L-Glutamin, 10 µg/ml Hydrokortison, 10 µg/ml Insulin, 10 µg/ml Penizillin und 10 µg/ml Streptomycin). Vor der Zugabe der zu testenden Substanzen wurden die Haarfollikel für 30 Minuten im Inkubator präinkubiert.

### c. Testung der Substanzen

Es werden Carnitin, Carnitintartrat sowie Minoxidil als Vergleichssubstanz mit bekannter Wirkung geprüft. Die Prüfkonzentration für Carnitin und Carnitiontartrat beträgt jeweils 50 µmol/l. Die Haarfollikel werden für 9 Tage in Kultur gehalten. Das Medium inklusive der Testsubstanzen wird täglich gewechselt. Die Länge der jeweiligen Haarschäfte wird am 9. Tag mit Hilfe eines Meßokulares am Dissektionsmikroskop gemessen und dokumentiert.

Die Prüfung von Minoxidil ist eine Positivkontrolle, weil Minoxidil in der Literatur bereits als wachstumsstimulierend für humane Haarfollikel beschrieben wird. Entsprechend dieser Literatur (Philpott, M.P., Green, M.R. and Kealey, T. in Human Hair Groth in Vitro, J. Cell. Sci. 97, S. 463 - 471, 1990) wird die Prüfung des Minoxidil durchgeführt. Hierzu sind folgende Änderungen gegenüber der Prüfung des Carnitines und des Carnitintartrates notwendig: Testkonzentration des Minoxidil 10⁻⁵ mol/l in 1% Ethanol, Ersatz des Streptomycines durch 10/µg/ml Ciprofloxacin. Zur statistischen Auswertung der Ergebnisse wird die Länge der Haarschäfte in % der Gesamtdurchschnittslänge aller Haarfollikel am Tage 0 berechnet Alle Haarfollikel einer Gruppe werden gepoolt und der Mittelwert sowie die Standardabweichung statistisch nach üblichen Methoden berechnet. Signifikante Unterschiede werden mit dem Student T- Test berechnet.

### d. Ergebnisse

**Tabelle 1: Ergebnisse zu Carnitin und Carnitintartrat:**

| Testsubstanz | Konzentration | Längenwachstum des Haarschaftes in % |
|---|---|---|
| Carnitin | 50 µmol/l | 67 |
| Carnitintartrat | 50 µmol/l | 87 |

**Tabelle 2: Positivkontrolle mit Minoxidil:**

| Testsubstanz | Konzentration | Längenwachstum des Haarschaftes in % |
|---|---|---|
| Minoxidil | 10⁻⁵ mol/l | 34 |
| Ohne Wirkstoff | | 18 |

### 2. Weitere Formulierungsbeispiele

### a) Haargel:

| | |
|---|---|
| Cetyl/Stearylalkohol + 30 EO | 19,0 |
| Oleylalkohol + 10 EO | 8,0 |
| Cetiol^{®} HE¹ | 15,0 |
| Cetiol^{®} LC² | 4,0 |
| Carnitintartrat | 4,0 |
| Tocopherylacetat | 0,4 |
| Panthenol | 2,0 |
| Glycerin | 4,0 |
| Parfümöl | 0,6 |
| Wasser | ad 100 |

| | |
|---|---|
| ¹ Polyolfettsaureester (CTFA-Bezeichnung: PEG-7-Glyceryl Cocoate) (HENKEL) ² Capryl/Caprinsäureester mit gesättigten Fettalkoholen C12-C18 (CTFA-Bezeichnung: Coco-Caprylate/Caprate) (HENKEL) | |

### b) Shampoo

| | |
|---|---|
| Natriumlauryl(EO)sulfat | 12,0 |
| Coco-Betaine | 5,0 |
| Carnitintartrat | 1,0 |
| Piroctone Olamine | 0,8 |
| Proteinhydrolysat auf pflanzlicher Basis | 0,5 |
| Parfümöl | 0,8 |
| Wasser | ad 100 |

## Patentansprüche

1. Kosmetisches Verfahren zur Verbesserung des Haarwuchses, **dadurch gekennzeichnet, daß** die Haare mit einem Mittel, enthaltend übliche Träger-, Wirk- und Hilfsstoffe behandelt werden, wobei dieses Mittel ein Mischsalz aus einem Betain der Formel (A-I) enthält,
R¹R²R³N⁺-(CR⁴R⁵)ₓ-(CR⁶R⁷)_{y}-(CR⁸R⁹)_{z}-Y⁻ (A-I)
in welchem das Betain der Formel (A-I) Camitin ist, und das Mischsalz als weitere Komponente Weinsäure enthält.

2. Kosmetisches Verfahren nach Anspruch 1, **dadurch gekennzeichnet, daß** das Mittel noch mindestens einen weiteren kosmetisch wirksamen Stoff enthält.

3. Kosmetisches Verfahren nach einem der Ansprüche 1 bis 2, **dadurch gekennzeichnet, daß** das Mittel einen Fettstoff (D) in Mengen von 0,1 bis 75 Gew.-%, bezogen auf das gesamte Mittel, enthält.

4. Kosmetisches Verfahren nach einem der Ansprüche 1 bis 3, **dadurch gekennzeichnet, daß** das Mittel ein Tensid (E) in Mengen von 0,1 bis 50 Gew.-%, bezogen auf das gesamte Mittel, enthält.

5. Kosmetisches Verfahren nach einem der Ansprüche 1 bis 4, **dadurch gekennzeichnet, daß** das Mittel einen Emulgator (F) in Mengen von 0,1 bis 25 Gew.-%, bezogen auf das gesamte Mittel, enthält.

6. Kosmetisches Verfahren nach einem der Ansprüche 1 bis 5, **dadurch gekennzeichnet, daß** das Mittel ein Polymer (G) in Mengen von 0,01 bis 10 Gew.-%, bezogen auf das gesamte Mittel, enthält.

7. Kosmetisches Verfahren nach einem der Ansprüche 1 bis 6, **dadurch gekennzeichnet, daß** das Mittel ein Proteinhydrolysat (H) in Mengen von 0,1 bis 10 Gew.-%, bezogen auf das gesamte Mittel, enthält.

8. Kosmetisches Verfahren nach einem der Ansprüche 1 bis 7, **dadurch gekennzeichnet, daß** das Mittel eine 2-Pyrrolidinon-2-carbonsäure und/oder deren Derivate (J) in Mengen von 0,01 bis 10 Gew.-%, bezogen auf das gesamte Mittel, enthält.

9. Kosmetisches Verfahren nach einem der Ansprüche 1 bis 8, **dadurch gekennzeichnet, daß** das Mittel ein Vitamin oder eine Vitaminvorstufe (K) in Mengen von 0,0001 bis 10 Gew.-%, bezogen auf das gesamte Mittel, enthält.

10. Kosmetisches Verfahren nach einem der Ansprüche 1 bis 9, **dadurch gekennzeichnet, daß** das Mittel einen Pflanzenextrakt (L) in Mengen von 0,01 bis 50 Gew.-%, bezogen auf das gesamte Mittel, enthält.

11. Kosmetisches Verfahren nach einem der Ansprüche 1 bis 10, **dadurch gekennzeichnet, daß** das Mittel einen pH-Wert von 2 bis 10 aufweist.

12. Verwendung von Mischsalzen aus Carnitin und Weinsäure zur Herstellung eines pharmazeutischen Mittels zur Förderung des Wachstumes und/oder Verhinderung des Ausfallens von Haaren.

## Claims

1. Cosmetic method for improving hair growth, **characterized in that** the hair is treated with an agent comprising customary carrier substances, active ingredients and auxiliaries, and a mixed salt of a betaine of the formula (A-I),
R¹R²R³N⁺-(CR⁴R⁵)ₓ-(CR⁶R⁷)_{y}-(CR⁸R⁹)_{z}-Y⁻ (A-I)
in which the betaine of the formula (A- I) is carnitine, and also **characterized in that** the mixed salt comprises tartaric acid as further component.

2. Cosmetic method according to Claim 1, **characterized in that** the agents also comprise at least one further cosmetically effective substance.

3. Cosmetic method according to one of Claims 1 or 2, **characterized in that** the agents comprise a fatty substance (D) in amounts of from 0.1 to 75% by weight, based on the total agent.

4. Cosmetic method according to one of Claims 1 to 3, **characterized in that** the agents comprise a surfactant (E) in amounts of from 0.1 to 50% by weight, based on the total agent.

5. Cosmetic method according to one of Claims 1 to 4, **characterized in that** the agents comprise an emulsifier (F) in amounts of from 0.1 to 25% by weight, based on the total agent.

6. Cosmetic method according to one of Claims 1 to 5, **characterized in that** the agents comprise a polymer (G) in amounts of from 0.01 to 10% by weight, based on the total agent.

7. Cosmetic method according to one of Claims 1 to 6, **characterized in that** the agents comprise a protein hydrolysate (H) in amounts of from 0.1 to 10% by weight, based on the total agent.

8. Cosmetic method according to one of Claims 1 to 7, **characterized in that** the agents comprise a 2- pyrrolidinone- 2- carboxylic acid and/or derivatives thereof (J) in amounts of from 0.01 to 10% by weight, based on the total agent.

9. Cosmetic method according to one of Claims 1 to 8, **characterized in that** the agents comprise a vitamin or a vitamin precursor (K) in amounts of from 0.0001 to 10% by weight, based on the total agent.

10. Cosmetic method according to one of Claims 1 to 9, **characterized in that** the agents comprise a plant extract (L) in amounts of from 0.01 to 50% by weight, based on the total agent.

11. Cosmetic method according to one of Claims 1 to 10, **characterized in that** the agents have a pH of from 2 to 10.

12. Use of mixed salts of carnitine and tartaric acid for preparing an pharmaceutical agent for promoting hair growth and/or preventing hair loss.

## Revendications

1. Procédé cosmétique pour améliorer la croissance des cheveux, **caractérisé en ce qu**'on traite les cheveux avec un agent contenant des supports, substances et adjuvants habituels et un sel mixte d'une bétaine de formule (A-I)
R¹R²R³N⁺-(CR⁴R⁵)ₓ-(CR⁶R⁷)_{y}-(CR⁸R⁹)_{z}-Y⁻ (A-I)
**caractérisés en ce que** la bétaine de formule (A-I) est la carnithine, et **caractérisés en outre en ce que** le sel mixte contient comme autre composant l'acide tartrique.

2. Procédé cosmétique selon la revendication 1, **caractérisés en ce que** les agents contiennent encore au moins une autre substance à action pharmacologique et/ou cosmétique.

3. Procédé cosmétique selon l'une des revendications 1 ou 2, **caractérisés en ce que** les agents contiennent un corps gras (D) en des quantités de 0,1 à 75 % en poids, par rapport à l'ensemble de l'agent.

4. Procédé cosmétique selon l'une des revendications 1 à 3, **caractérisés en ce que** les agents contiennent un agent tensioactif (E) en des quantités de 0,1 à 50 % en poids, par rapport à l'ensemble de l'agent.

5. Procédé cosmétique selon l'une des revendications 1 à 4, **caractérisés en ce que** les agents contiennent un émulsifiant (F) en des quantités de 0,1 à 25 % en poids, par rapport à l'ensemble de l'agent.

6. Procédé cosmétique selon l'une des revendications 1 à 5, **caractérisés en ce que** les agents contiennent un polymère (G) en des quantités de 0,01 à 10 % en poids, par rapport à l'ensemble de l'agent.

7. Procédé cosmétique selon l'une des revendications 1 à 6, **caractérisés en ce que** les agents contiennent un hydrolysat de protéine (H) en des quantités de 0,1 à 10 % en poids, par rapport à l'ensemble de l'agent.

8. Procédé cosmétique selon l'une des revendications 1 à 7, **caractérisés en ce que** les agents contiennent un acide 2- pyrrolidinone- 2- carboxylique et/ou ses dérivés (J) en des quantités de 0,01 à 10 % en poids, par rapport à l'ensemble de l'agent.

9. Procédé cosmétique selon l'une des revendications 1 à 8, **caractérisés en ce que** les agents contiennent une vitamine ou un précurseur de vitamine (K) en des quantités de 0,0001 à 10 % en poids, par rapport à l'ensemble de l'agent.

10. Procédé cosmétique selon l'une des revendications 1 à 9, **caractérisés en ce que** les agents contiennent un extrait de plante (L) en des quantités de 0,01 à 50 % en poids, par rapport à l'ensemble de l'agent.

11. Procédé cosmétique selon l'une des revendications 1 à 10, **caractérisés en ce que** les agents présentent un pH allant de 2 à 10.

12. Utilisation de sels mixtes de carnithine et d'acide tartrique pour la production d'un agent pharmaceutiques visant à favoriser la croissance et/ou à empêcher la chute des cheveux.
